Europäisches Patentamt

European Patent Office

Office européen des brevets

(19)

(11) EP 0 556 424 B1

(12)

# FASCICULE DE BREVET EUROPEEN

(45) Date de publication et mention
de la délivrance du brevet:
**20.05.1998 Bulletin 1998/21**

(51) Int Cl.⁶: **C11B 3/10**, A61K 7/48,
A23C 11/00, A23L 1/30

(21) Numéro de dépôt: **92102742.1**

(22) Date de dépôt: **19.02.1992**

(54) **Procédé de décoloration d'ester d'acide gras et composition alimentaire ou cosmétique le contenant**

Verfahren zum Entfärben von Fettsäureester und Nahrungsmittel- oder kosmetische Zubereitung diesen enthaltend

Process for decolorizing fatty acid ester and food or cosmetic composition containing the same

(84) Etats contractants désignés:
**AT BE CH DE DK ES FR GB GR IT LI LU NL PT SE**

(43) Date de publication de la demande:
**25.08.1993 Bulletin 1993/34**

(73) Titulaire: **SOCIETE DES PRODUITS NESTLE S.A.**
**1800 Vevey (CH)**

(72) Inventeurs:
• **Gonus, Philippe**
**CH-1608 Chesalles sur Oron (CH)**
• **Wille, Hans-Jürgen**
**CH-1844 Villeneuve (CH)**

(56) Documents cités:
**EP-A- 0 079 799          EP-A- 0 295 418**
**FR-A- 2 019 604          FR-A- 2 482 867**
**US-A- 3 369 993**

• **FETT WISSENSCHAFT TECHNOLOGIE- FAT SCIENCE TECHNOLOGY vol. 93, no. 04, 1991, LEINFELDEN ECHTERDINGEN DE pages 132 - 135 F. CHO-AH-YING ET AL. 'Adsorptive removal of sulfur from canola oil'**
• **LA RIVISTA ITALIANA DELLE SOSTANZE GRASSE vol. 67, no. 05, 1990, IT pages 257 - 258 L. ANGELIN ET AL. 'Oil extraction and refining from Somaly Papaya seeds'**

## Description

L'invention concerne un procédé de décoloration d'ester d'acide gras, notamment d'huile spéciale, destinés à entrer dans la composition de produits alimentaires ou cosmétiques.

Les huiles brutes extraites par pressage ou au moyen de solvants ne peuvent pas, dans la plupart des cas, être utilisées telles quelles dans les produits alimentaires ou cosmétiques. Elles contiennent des impuretés qui doivent être éliminées par un raffinage préalable. Le raffinage se déroule généralement en quatre étapes successives: le dégommage à l'acide, la neutralisation alcaline, la décoloration et enfin la désodorisation.

Dans certains cas particuliers d'huiles fortement colorées destinées à des applications spécifiques, par exemple en cosmétique nécessitant une décoloration poussée, le raffinage classique consistant en une mise en contact avec de la bentonite ou de la montmorillonite activée à l'acide à chaud et sous vide, puis une filtration ne permet pas d'atteindre le niveau de décoloration requis pour l'application envisagée.

Un procédé amélioré connu de décoloration dit "chromatographique", consiste à diluer l'huile dans un solvant apolaire et à adsorber les impuretés par contact de la solution avec un adsorbant solide dans une colonne. Un tel procédé est décrit par exemple dans la demande de brevet européenne EP-A-0108571 relative en particulier à la stabilisation et à la décoloration des huiles de poisson . Selon ce procédé, on hydrogène une huile de poisson riche en acides gras polyinsaturés en présence d'un catalyseur, puis on la décolore en solution au moyen d'un adsorbant solide tel que le silicagel, l'alumine activée, le silicate d'aluminium ou l'argile activée, par passages successifs de la solution à travers une colonne contenant l'adsorbant.

EP-A-0079799 concerne un procédé de décoloration de beurre végétal tropical, de Sal, d'Illipé ou de Shea en vue d'une utilisation comme substitut de beurre de cacao. Ce procédé, par charges, comporte 3 étapes: La mise en présence de la graisse dissoute dans l'hexane à titre de solvant et d'une terre décolorante, la filtration pour séparer l'adsorbant, puis le passage de la solution à travers une colonne chargée de silicagel. Pour ce type de beurre végétal, assez peu sensible à l'oxydation, un procédé en plusieurs étapes comprenant plusieurs manipulations peut être acceptable.

Un inconvénient de ce procédé est cependant qu'il est discontinu, puisqu'il nécessite la combinaison d'un traitement en dispersion en cuve sous agitation avec la terre décolorante suivi d'une séparation avant un traitement en continu dans une colonne. De plus, il ne peut pas y avoir d'interaction des adsorbants par combinaison de leurs effets, puisque les traitements sont successifs.

FR-A-2019604 a trait à un procédé de préparation d'un adsorbant à base de terre décolorante destiné au traitement d'huiles végétales, animales ou minérales, en colonne, par passage direct, sans solvant, du corps gras à traiter. Comme expliqué, par exemple à la page 1, lignes 11-37 de l'antériorité, la terre décolorante devait être finement divisée pour être efficace, ce qui n'allait pas sans risque de détérioration du corps gras lors de la filtration à chaud en présence d'air et ce problème a été résolu en activant et en agrégant les particules d'adsorbant pour former des granules. L'agrégation des particules d'adsorbant est sensée conférer aux granules une bonne porosité et une bonne résistance à l'écrasement, ce qui les rend aptes au traitement en colonne, alors que le produit en poudre conduirait au blocage de la colonne.

Nous avons trouvé de manière inattendue que l'utilisation d'un mélange adsorbant d'argile montmorillonite et de silicagel ou de charbon actif dans un procédé de décoloration par passage sur colonne d'adsorbant permettait de décolorer pratiquement complètement des huiles jusqu'alors impossibles à décolorer à un degré suffisant avec les procédés de décoloration usuels mentionnés précédemment.

Le procédé selon l'invention, dans lequel on fait passer un ester d'acide gras en solution dans un solvant apolaire à travers une colonne remplie d'adsorbant, puis on élimine le solvant, est caractérisé par le fait que l'on utilise un mélange à base de montmorillonite contenant du silicagel et/ou du charbon actif à titre d'adsorbant.

De préférence, l'adsorbant peut être constitué d'un mélange contenant 10 à 60 % en poids de montmorillonite et jusqu'à 80 % en poids de silicagel.

Il peut contenir uniquement ces deux constituants, par exemple environ 50% en poids de silicagel et environ 50% en poids de montmorillonite.

Dans une variante particulièrement préférée, l'adsorbant contient en sus 30 à 60 % en poids de charbon actif.

Dans une autre forme de réalisation particulièrement avantageuse, le mélange adsorbant contient 30 à 80% en poids de charbon actif et 20 à 70% en poids de montmorillonite.

La montmorillonite peut être sous forme de poudre dont la dimension des particules est par exemple de 5 à 60 $\mu$m (micron) ou de granulés par exemple de 60 à 250 $\mu$m (micron).

Le silicagel peut se présenter sous forme de granulés de diamètre, par exemple 60 à 500 $\mu$m (micron).

Le charbon actif peut être constitué de granulés de diamètre, par exemple 100 à 500 $\mu$m (micron) avec des pores, par exemple de quelques microns constituant des alvéoles à la surface des granulés.

La montmorillonite peut se présenter sous forme de poudre ou de granulés ou d'un mélange contenant de préférence 40 à 60% en poids de poudre et 60 à 40% en poids de granulés.

Le mélange adsorbant peut en outre contenir un élément de garnissage à titre d'adjuvant de filtration, par exemple une terre de diatomées, par exemple la diatomite ou la perlite allant jusqu'à 30 % de son poids.

Les composés à décolorer par la mise en oeuvre du procédé selon l'invention peuvent être des huiles animales, par exemple de poisson ou végétales, par exemple de graines de cumin, de Lesquerella, d'Hévéa brasiliensis, de sureau, de Calendula, de kiwi, de myrtille, de café ou d'origine biosynthétique, par exemple d'enveloppes de levure ou encore d'origine synthétique, par exemple un butanediol diester d'acides gras.

Pour mettre en oeuvre le procédé, on met le composé à traiter préalablement raffiné de manière classique par dégommage, neutralisation et décoloration comme indiqué précédemment, en solution dans un solvant apolaire, par exemple un hydrocarbure aliphatique, de préférence le n-hexane qui est de qualité alimentaire, dans un rapport pondéral composé: solvant de 1:2 à 1:16 et de préférence environ 1:9. La mise en solution peut nécessiter un chauffage du composé pour le liquéfier et la solution doit être limpide. Le rapport composé: solvant dépend de la nature du composé à traiter et résulte d'un compromis entre une quantité minimale de solvant qu'il faudra ensuite éliminer et une dilution suffisante du composé pour que la solution soit peu polaire.

On fait ensuite passer la solution dans une colonne remplie du mélange adsorbant par gravimétrie en utilisant de préférence un rapport pondéral corps gras: adsorbant de 0,5:1 à 5:1, par exemple 2:1 et en appliquant une pression basse ou moyenne, par exemple jusqu'à 1,5 b selon le type de colonne et la granulométrie de l'adsorbant utilisés.

On peut utiliser entre autres des colonnes de diamètre 27 à 225 mm et de hauteur 10 à 100 cm, les colonnes courtes et de relativement grand diamètre étant préférées, les colonnes dont le rapport diamètre: hauteur est inférieur à 1:2 permettant l'emploi d'un adsorbant de granulométrie fine, le travail à basse pression, par exemple inférieure à environ 0,2 b étant préféré. Le temps de passage de la solution à travers la colonne est par exemple de 15 à 60 min par litre de solution pour environ 50 g d'adsorbant.

Après percolation, on rince la colonne avec du solvant, de préférence avec un volume de solvant correspondant à environ 25 % de celui de la solution.

Après avoir réuni les liquides, on élimine le solvant, par exemple par évaporation sous vide.

Une composition alimentaire ou cosmétique pourra contenir un composé décoloré par la mise en oeuvre du procédé selon l'invention.

Une composition cosmétique contiendra par exemple les huiles décolorées de cumin, Calendula, myrtille, sureau, café, kiwi, Hevea brasiliensis ou un butanediol diester dans une crème, l'huile de Lesquerella dans un rouge à lèvres, intéressants pour leurs propriétés cosmétiques.

Une composition alimentaire diététique contiendra par exemple les huiles décolorées de myrtille, sureau, kiwi ou de poisson comme source d'acides gras de la série omega 3. Une composition diététique, utilisable en diététique humaine ou animale pourra contenir par exemple un butanediol diester décoloré à titre de substitut de corps gras non assimilable par l'organisme.

Les exemples ci-après illustrent l'invention.

Dans ceux-ci:

- les pourcentages et parties sont pondéraux sauf indication contraire.
- Les analyses effectuées pour déterminer la qualité des produits et le niveau de la décoloration sont les suivantes:

FFA: acides gras libres exprimés en % d'acide oléique;
POV: indice de peroxyde en milliéquivalent g d'oxygène/kg
Y(jaune), R(rouge), N(neutre): unités colorimétriques mesurées avec l'échelle Lovibond (cuve de 12,7; 25,4 ou 133,35 mm de trajet optique).

- Les conditions expérimentales valables pour tous les exemples sont les suivantes:
On prépare l'adsorbant en mélangeant à sec ses constituants jusqu'à l'obtention d'un mélange homogène dans un récipient pouvant être mis sous vide. On ajoute le solvant et on mélange vigoureusement tout en appliquant pendant 2 à 3 min un vide de 15 à 30 mb, par exemple au moyen d'une trompe à eau, de manière à dégazer l'adsorbant dont la masse volumique apparente est environ 0,4 à 0,5 kg/dm$^3$.

Pour préparer la colonne, qui est munie d'une plaque filtrante à sa base, on y introduit d'abord du n-hexane pour la purger, puis on la remplit avec l'adsorbant en suspension dans le n-hexane.

On dilue l'huile ou la graisse, préalablement fondue le cas échéant, dans le n-hexane, de manière à former une solution parfaitement limpide.

On percole alors la solution à travers la colonne, le cas échéant en appliquant une pression de 0,2 à 1,5 b (exprimé par rapport à la pression ambiante) pendant 15 à 60 min avec 1 1 de solution pour 50 g d'adsorbant selon le type de colonne, puis on rince la colonne avec un volume de n-hexane correspondant au 1/4 du volume de solution à décolorer. Après avoir réuni les liquides, on élimine le n-hexane par évaporation sous vide dans un évaporateur rotatif. Le n-hexane peut être recyclé et utilisé pour une nouvelle mise en solution du corps gras.

Les adsorbants utilisés sont les suivants:

a: Silicagel de granulométrie 200-500 µm
b: Montmorillonite en poudre, 5-50 µm
c: Montmorillonite granulée, 150-250 µm
d: silicagel granulé, 63-200 µm
e: Silicagel granulé, 100-400 µm
f: Charbon actif granulé, 100-500 µm avec alvéoles de quelques µm en surface
g: Terre de diatomées, 10-100 µm

La décoloration lors du raffinage classique correspond à la mise en contact de la solution avec 0,5-3% en poids de montmorillonite activée sous vide de 1-2 mb, à une température de 80-100°C, puis à la filtration sur filtre-presse.

Exemples 1-2

Les paramètres du procédé et les résultats sont indiqués dans le tableau 1 ci-après pour l'huile de graines de cumin dont la composition en acides gras est:

| Acides gras | % |
|---|---|
| C16:0 | 4,3 |
| C18:0 | 1,1 |
| C18:1,$\Delta$6 (pétrosélénique) et $\Delta$9 | 59,9 |
| C18:2 | 33,4 |
| Autres | 1,3 |

Tableau 1

| Exemple | Comparaison 1 | Comparaison 2 | 1 | 2 |
|---|---|---|---|---|
| Prise (g) | après décoloration conventionnelle | 150 | 150 | 150 |
| n-Hexane (ml) | | 450 | 450 | 450 |
| Adsorbant (type) | | a | a+b | a+b+c |
| Proportions (%) des composants de l'adsorbant | | 100 | a:80 | a:50 |
| | | | b:20 | b:20 |
| | | | | c:30 |
| Quantité (g) | | 75 | 75 | 75 |
| Type de colonne | | verre | verre | verre |
| Diamètre (mm) | | 27 | 27 | 27 |
| Hauteur (cm) | | 25 | 25 | 25 |
| Pression (b) | | ambiante | 0,2 | 0,2 |
| FFA (% acide oléique) | 0,1 | 0,05 | 0,05 | 0,04 |
| POV (méq0$_2$/kg) | 0,86 | 0,75 | 0,26 | 0,33 |
| Couleur Y | 68 | 39 | 3,7 | 3,6 |
| (Cuve 25,4 mm) R | 8,8 | 3,4 | 1 | 0,8 |

On constate que le traitement par le mélange adsorbant silicagel/montmorillonite permet une meilleure élimination des peroxydes et apporte une amélioration considérable de la décoloration par rapport au produit de départ ayant subi une décoloration conventionnelle (comparaison 1) et par rapport à l'utilisation du silicagel seul (comparaison 2).

Par ailleurs, la décoloration conventionnelle est extrémement lente, ce qui est dû à une prise en masse de la montmorillonite en présence de solvant.

Exemples 3-7

Les paramètres du procédé et les résultats sont indiqués dans le tableau 2 ci-après pour l'huile de graines de Lesquerella dont la composition en acides gras est indiquée ci-après.

| Acides gras | % |
|---|---|
| C16:0 | 2,1 |
| C16:1 | 1,5 |
| C18:0 | 3,2 |
| C18:1 | 23,2 |
| C18:2 | 9,7 |
| C18:3 $\alpha$ | 15,1 |
| C20:1 | 1,5 |
| C20:1 (14-OH), lesquerolique | 43,3 |
| Autres | 0,4 |

Cette huile a la particularité de contenir une grande quantité d'acide lesquérolique, hydroxylé, qui la rend difficile à décolorer par la voie conventionnelle.

## Tableau 2

| Exemple | Comparaison 3 | Comparaison 4 | 3 | 4 |
|---|---|---|---|---|
| Prise (g) | | | 50 | 50 |
| n-Hexane (ml) | après | après | 500 | 800 |
| Adsorbant (type) | 1 | 2 | a+b+c | a+b+c+d |
| Proportions (%) | | | a:50 | a:25 |
| des composants | déco- | déco- | b:20 | b:25 |
| de l'adsorbant | lora- | lora- | c:30 | c:25 |
| | tion | tions | | d:25 |
| Quantité (g) | | | 50 | 50 |
| d'adsorbant | con- | con- | | |
| Type de colonne | ven- | ven- | verre | verre |
| Diamètre (mm) | tion- | tion- | 27 | 27 |
| Hauteur (cm) | nelle | nelles | 25 | 25 |
| Pression (b) | | | 0,2 | 0,2 |
| Couleur          Y | 68 | 69 | 35,5 | 17,4 |
| (Cuve 12,7 mm) R | 12,6 | 11,9 | 1,4 | 1,1 |
| N | 2,5 | 2,6 | 0,2 | 0,7 |

## Tableau 2 (suite)

| Exemple | 5 | 6 | 7 |
|---|---|---|---|
| Prise (g) | 50 | 50 | 515 |
| Hexane (ml) | 500 | 500 | 5000 |
| Adsorbant (type) | a+b+c+d | e+b | d+b |
| Proportions (%) | a:25 | e:50 | d:50 |
| des composants | b:25 | b:50 | b:50 |
| de l'adsorbant | c:25 | | |
| | d:25 | | |
| Quantité (g) d'adsorbant | 50 | 50 | 600 |
| Type de colonne | verre | verre | acier |
| Diamètre (mm) | 50 | 50 | 125 |
| Hauteur (cm) | 10 | 10 | 15 |
| Pression (b) | ambiante | ambiante | 0,3 |
| Couleur        Y | 13 | 9,9 | 8,6 |
| (Cuve 12,7 mm) R | 1,0 | 0,9 | 0,6 |
| N | 0,3 | 0 | 0,2 |

On constate que la décoloration conventionnelle ne permet pas d'améliorer la décoloration malgré deux traitements successifs (comparaisons 3 et 4).

Dans les exemples 3-7, la matière première mise en oeuvre est une huile ayant subi deux décolorations conventionnelles successives et celle-ci est considérablement décolorée par un seul passage.

Exemples 8-11

Ces exemples concernent la décoloration de l'huile d'hévea brasiliensis dont la composition en acides gras est indiquée ci-après.

| Acides gras | % |
|---|---|
| C16:0 | 8,8 |
| C18:0 | 8,7 |
| C18:1 | 24,9 |
| C18:2 | 38,6 |

7

(suite)

| Acides gras | % |
|---|---|
| C18:3 α | 16,7 |
| Autres | 2,3 |

Les conditions opératoires et les résultats sont indiqués dans les tableaux 3 et 4 ci-après.

Tableau 3

| Exemple | Comparaison 5 | Comparaison 6 | 8 | 9 |
|---|---|---|---|---|
| Prise (g) | | 150 | 150 | 150 |
| n-Hexane (ml) | après | 450 | 450 | 450 |
| Adsorbant (type) | 1 | a | a+b | a+b+c |
| Proportions (%) des composants de l'adsorbant | décoloration conventionnelle | 100 | a:80 | a:50 |
| | | | b:20 | b:20 |
| | | | | c:30 |
| Quantité (g) d'adsorbant | | 75 | 75 | 75 |
| Type de colonne | | verre | verre | verre |
| Diamètre (mm) | | 27 | 27 | 27 |
| Hauteur (cm) | | 25 | 25 | 25 |
| Pression (b) | | ambiante | 0,2 | 0,2 |
| POV (méq0$_2$/kg) | 0,66 | 1,05 | 0,64 | 0,96 |
| Couleur Y | 68 | 24,5 | 2,2 | 2,2 |
| (Cuve 25,4 mm) R | 13,3 | 3,9 | 0,6 | 0,5 |

Tableau 4

| Exemple | Compa-raison 7 | compa-raison 8 |
|---|---|---|
| Prise (g) | 100 | 100 |
| n-Hexane (ml) | 900 | 900 |
| Adsorbant (type) | b | f |
| Proportions (%) | 100 | 100 |
| des composants | | |
| de l'adsorbant | | |
| Quantité (g) | 50 | 50 |
| Type de colonne | verre | verre |
| Diamètre (mm) | 50 | 50 |
| Hauteur (cm) | 10 | 10 |
| Pression (b) | 0,2 | ambiante |
| Couleur          Y | 6,1 | 3,1 |
| (Cuve 25,4 mm) R | 1,5 | 0,9 |

## Tableau 4 (suite)

| Exemple | 10 | 11 |
|---|---|---|
| Prise (g) | 100 | 100 |
| n-Hexane (ml) | 900 | 900 |
| Adsorbant (type) | a+b+c | d+b+f |
| Proportions (%) | a:50 | d:33,3 |
| des composants | b:20 | b:33,3 |
| de l'adsorbant | c:30 | f:33,3 |
| Quantité (g) d'adsorbant | 50 | 50 |
| Type de colonne | verre | verre |
| Diamètre (mm) | 50 | 50 |
| Hauteur (cm) | 10 | 10 |
| Pression (b) | ambiante | ambiante |
| Couleur            Y | 2,4 | 0,5 |
| (Cuve 25,4 mm) R | 0,7 | 0 |

On constate que le procédé de décoloration avec un mélange d'adsorbants selon l'invention procure des résultats nettement meilleurs (exemples 8 et 9 rapprochés de comparaison 6), (exemple 10 rapproché de comparaison 7), (exemple 11 rapproché de comparaisons 7 et 8), que ceux obtenus par mise en oeuvre de l'un des adsorbants seul dans les mêmes conditions opératoires.

Par ailleurs, les mélanges d'adsorbants permettent une décoloration poussée qui n'est pas réalisable par l'utilisation de décoloration conventionnelle (comparaison 5).

Exemples 12-15

Les paramètres du procédé et les résultats relatifs à la décoloration de l'huile de pépins de kiwi sont indiqués dans le tableau 5 ci-après.

L'huile mise en oeuvre a la composition en acides gras indiquée ci-après.

| Acides gras | % |
|---|---|
| C16:0 | 4,9 |
| C18:0 | 2,6 |

(suite)

| Acides gras | % |
|---|---|
| C18:1 | 11,2 |
| C18:2 | 14,9 |
| C18:3 $\alpha$ | 63,8 |
| Autres | 2,6 |

Tableau 5

| Exemple | Comparaison 9 | 12 | 13 | 14 | 15 |
|---|---|---|---|---|---|
| Prise (g) | | 200 | 200 | 200 | 15000 |
| n-Hexane (ml) | après | 1800 | 1800 | 1800 | 135000 |
| Adsorbant | 1 | | | | |
| b (%) | | 50 | 25 | 60 | 55 |
| f (%) | décoloration conventionnelle | 50 | 75 | 40 | 45 |
| Quantité (g) d'adsorbant | | 50 | 50 | 50 | 5000 |
| Type de colonne | | verre | verre | verre | verre |
| Diamètre (mm) | | 50 | 50 | 50 | 225 |
| Hauteur (cm) | | 10 | 10 | 10 | 80 |
| Pression (b) | | ambiante | ambiante | ambiante | ambiante |
| Couleur Y | 68 | 9,6 | 11,2 | 7,3 | 5,4 |
| (Cuve 133,35 mm) R | 8,1 | 1,2 | 1,3 | 1,0 | 0,8 |
| N | 1,7 | 0,2 | 0,6 | 0,2 | 0,2 |

On constate que le mélange adsorbant montmorillonite/charbon actif permet une décoloration substantielle d'une huile de pépins de kiwi très colorée qui ne peut pas être atteinte à ce degré par la voie conventionnelle (comparaison 9).

Exemples 16-19

On procède à la décoloration d'huile de myrtilles (16), de sureau (17), de calendula (18) et de poisson (19) dans les conditions et avec les résultats indiqués dans le tableau 6 ci-après.
Ces huiles ont les compositions en acides gras indiquées ci-après.

| Acides gras | % | | | |
|---|---|---|---|---|
| | 16 | 17 | 18 | 19 |
| C16:0 | 4,1 | 7,4 | 3,2 | 17,6 |
| C16:1 | -- | -- | -- | 4,7 |
| C18:0 | 1,1 | 2 | 2,1 | 4,5 |
| C18:1 | 23,2 | 14 | 4,9 | 21,8 |
| C18:2 | 35,9 | 41 | 31,3 | 1,4 |
| C18:3 $\alpha$ | 34,3 | 30,1 | 0,6 | 0,4 |
| C18:3 $\Delta$ 8,10,12 | -- | -- | 48 | -- |
| C20:1 | -- | -- | -- | 2,5 |
| C20:5 | -- | -- | -- | 6,2 |
| C22:6 | -- | -- | -- | 23,9 |

(suite)

| Acides gras | % | | | |
|---|---|---|---|---|
| | 16 | 17 | 18 | 19 |
| Autres | 1,3 | 5,5 | 9,9 | 17 |

Tableau 6

| Exemple | 16 | 17 | 18 | 19 |
|---|---|---|---|---|
| Prise (g) | 650 | 650 | 1000 | 100 |
| n-Hexane (ml) | 2600 | 2600 | 3000 | 400 |
| Adsorbant (type) | a+b+g | a+b+c | a+b+c | a+b+g |
| Proportions (%) des composants de l'adsorbant | a:70 | a:60 | a:60 | a:60 |
| | b:20 | b:20 | b:20 | b:20 |
| | g:10 | c:20 | c:20 | g:20 |
| Quantité (g) d'adsorbant | 330 | 330 | 660 | 50 |
| Type de colonne | verre | verre | verre | verre |
| Diamètre (mm) | 27 | 35 | 50 | 27 |
| Hauteur (cm) | 25 | 55 | 100 | 25 |
| Pression (b) | 0,2 | 0,8 | 1,5 | 0,2 |
| Couleur Y | 0,3 | 0,5 | 12,5 | 0,8 |
| (Cuve 25,4 mm) R | 0,1 | 0,05 | 0,6 | 0,1 |
| Comparaison 10, couleur après Y | 31 | 7 | >40 | 24 |
| 1 décoloration conventionnelle R (Cuve 25,4 mm) | 5,3 | 1 | 6,8 | 2,4 |

On constate une nette amélioration de la décoloration par la mise en oeuvre selon l'invention d'un mélange adsorbant par rapport à une décoloration conventionnelle.

Exemple 20

Les paramètres du procédé et les résultats de la décoloration de 2,3-butanedioldiester préparé selon l'exemple 1 de la demande de brevet EP 0465689 sont indiqués dans le tableau 7 ci-après.

Tableau 7

| Exemple | Comparaison 11 | 20 |
|---|---|---|
| Prise (kg) | | 21 |
| n-Hexane (1) | 1 | 84 |

Tableau 7 (suite)

| Exemple | Comparaison 11 | 20 |
|---|---|---|
| Adsorbant (type) | décoloration conventionnelle | f+b |
| Proportions (%) des composants de l'adsorbant | | f: 55 |
| | | b: 45 |
| Quantité (kg) d'adsorbant | | 8,4 |
| Type de colonne | | verre |
| Diamètre (mm) | | 225 |
| Hauteur (cm) | | 80 |
| Débit (1/h) | | 23 |
| Couleur | | |
| Cuve Y | 23,3 | --- |
| (25,4 mm) R | 4,9 | --- |
| B | 0,5 | --- |
| Cuve Y | 37,8 | 0,8 |
| (133,4 mm) R | 52,8 | 0,1 |
| B | 32 | 0 |

On constate que la décoloration est pratiquement totale par la mise en oeuvre du procédé selon l'invention. Par contre, une décoloration conventionnelle ne permet pas d'atteindre le résultat.

Exemples 21-22

Dans ces exemples, la nomenclature utilisée est celle de la "Cosmetic, Toiletery and Flagrance Association, Inc. Washington DC" (CFTA), mise à part la traduction en français.

21. Pour fabriquer une crème cosmétique sous forme d'une émulsion huile-dans-l'eau, on mélange séparément les composants de phases lipidiques A et a et on les chauffe à 70°C, puis on incorpore la phase B dans la phase A. On prépare la phase aqueuse C en mélangeant ses composants et on la chauffe à 70°C. On ajoute les phases lipidiques A et B à la phase aqueuse C à 70°C en brassant à vitesse moyenne. On homogénéise le mélange des deux phases puis on le brasse à environ 100 t/min et on le laisse refroidir à 35-40°C.

A cette température, on ajoute les additifs, puis on continue à refroidir jusqu'à la température ambiante en brassant lentement et on arrête le brassage lorsque le produit est semi-fluide (à environ 25°C).

La crème a la composition suivante:

| | | % |
|---|---|---|
| Phase A (lipidique) | | 12,1 |
| PEG-10 isocétyl éther monostéarate | 4,5 | |
| Stéareth-21 | 1,5 | |
| Stéarate de glycérol | 2,6 | |
| Alcool cétéarylique | 1,5 | |
| Isodécyl laurate | 2 | |
| Phase B (lipidique) | | 6,3 |
| Huile de cumin décolorée | | |

(suite)

|  | % |  |
|---|---|---|
| de l'exemple 1 | 6 |  |
| Carbomer 934 (polymère d'acide acrylique polyréticulé) | 0,3 |  |
| Phase C (aqueuse) |  | 79,8 |
| Eau | 74,7 |  |
| Glycérol | 5 |  |
| Ethylènediaminetétraacétate (EDTA) | 0,1 |  |
| Additifs |  | 1,8 |
| Phénoxyparaben | 0,6 |  |
| Silméthicone | 0,1 |  |
| Triméthamine (solution aqueuse à 30%) | 0,8 |  |
| Parfum | 0,3 |  |
|  |  | 100 |

22. On utilise l'huile de Lesquerella décolorée en remplacement de l'huile de ricin dans un rouge à lèvres, anhydre.

Le produit est obtenu de la même manière que dans l'exemple 21, mais sans homogénéisation, par mélange à chaud (environ 70°C), puis refroidissement progressif sous brassage lent.

Il y a la composition suivante:

|  | % |
|---|---|
| Huile de Lesquerella décolorée selon l'exemple 7 | 27,45 |
| Huile de castor | 30,5 |
| Cire d'abeilles | 10,5 |
| Cire de candelilla | 7,5 |
| Ozokérite | 5,5 |
| Lanolate d'isopropyle | 5 |
| Colorants | 13,55 |

## Exemple 23

On prépare un produit laitier infantile à reconstituer par addition d'eau sous forme de poudre par mélange des phases aqueuses et lipidique, concentration par évaporation et séchage par pulvérisation en tour dans des conditions ménagées.

La composition des matières sèches est la suivante:

| Ingrédients | % |
|---|---|
| Mélange de graisses contenant de la lécithine | 26 |
| Huile de poisson décolorée selon l'exemple 19 | 0,6 |
| Lactose et maltodextrine | 60 |
| Protéines lactiques | 11 |
| Sels minéraux | 1,6 |
| Vitamines et oligoéléments | 0,8 |
|  | 100 |

14

## Revendications

1. Procédé de décoloration d'ester d'acide gras, notamment d'huile spéciale fortement colorée destinée à un usage alimentaire ou cosmétique, dans lequel on fait passer un ester d'acide gras en solution dans un solvant apolaire à travers une colonne remplie d'adsorbant, puis on élimine le solvant, caractérisé par le fait que l'on utilise un mélange à base de montmorillonite contenant du silicagel et/ou du charbon actif à titre d'adsorbant.

2. Procédé selon la revendication 1, caractérisé par le fait que l'on met en oeuvre un mélange adsorbant contenant 10 à 60% en poids de montmorillonite et jusqu'à 80% en poids de silicagel.

3. Procédé selon la revendication 2, caractérisé par le fait que le mélange adsorbant contient en sus 30 à 60% en poids de charbon actif.

4. Procédé selon la revendication 1, caractérisé par le fait que l'on met en oeuvre un mélange adsorbant contenant 30 à 80% en poids de charbon actif et 20 à 70% en poids de montmorillonite.

5. Procédé selon la revendication 3 ou 4, caractérisé par le fait que le charbon actif est sous forme de granulés et la montmorillonite sous forme de poudre.

6. Procédé selon la revendication 2, caractérisé par le fait que le mélange adsorbant contient environ 50% en poids de silicagel et environ 50% en poids de montmorillonite.

7. Procédé selon la revendication 2 ou 6, caractérisé par le fait que la montmorillonite est constituée de 40 à 60% en poids de poudre et 60 à 40% en poids de granulés.


## Patentansprüche

1. Verfahren zur Entfärbung von Fettsäureester, insbesondere eines stark gefärbten Spezialöls für eine Verwendung in Nahrungsmitteln oder in der Kosmetik, bei dem man einen Fettsäureester in Lösung in einem apolaren Lösungsmittel über eine Säule mit einem Adsorptionsmittel leitet und dann das Lösungsmittel entfernt, dadurch gekennzeichnet, daß man als Adsorptionsmittel eine Mischung auf der Basis von Montmorillonit verwendet, die Kieselgel und/oder Aktivkohle enthält.

2. Verfahren nach Anspruch 1, dadurch gekennzeichnet, daß man eine Adsorptionsmittelmischung einsetzt, die 10 bis 60 Gew.-% Montmorillonit und bis zu 80 Gew.-% Kieselgel enthält.

3. Verfahren nach Anspruch 2, dadurch gekennzeichnet, daß die Adsorptionsmittelmischung außerdem 30 bis 60 Gew.-% Aktivkohle enthält.

4. Verfahren nach Anspruch 1, dadurch gekennzeichnet, daß man eine Adsorptionsmittelmischung einsetzt, die 30 bis 80 Gew.-% Aktivkohle und 20 bis 70 Gew.-% Montmorillonit enthält.

5. Verfahren nach Anspruch 3 oder 4, dadurch gekennzeichnet, daß die Aktivkohle in Form eines Granulats vorliegt und der Montmorillonit in Form eines Pulvers.

6. Verfahren nach Anspruch 2, dadurch gekennzeichnet, daß die Adsorptionsmittelmischung etwa 50 Gew.-% Kieselgel und etwa 50 Gew.-% Montmorillonit enthält.

7. Verfahren nach Anspruch 2 oder 6, dadurch gekennzeichnet, daß der Montmorillonit von 40 bis Gew.-% Pulver und 60 bis 40 Gew.-% Granulat gebildet wird.


## Claims

1. Process for decolorizing a fatty acid ester, in particular a strongly coloured special oil intended for food or cosmetic use, wherein a fatty acid ester dissolved in a non-polar solvent is passed through a column filled with adsorbent and the solvent is then removed,

characterized in that a mixture is used based on montmorillonite containing silica gel and/or activated carbon as an adsorbent.

2. Process according to claim 1, characterized in that an adsorbent mixture is used containing 10 to 60 % by weight of montmorillonite and up to 80 % by weight of silica gel.

3. Process according to claim 2, characterized in that the adsorbent mixture additionally contains 30 to 60 % by weight of activated carbon.

4. Process according to claim 1, characterized in that an adsorbent mixture is used containing 30 to 80 % by weight of activated carbon and 20 to 70 % by weight of montmorillonite.

5. Process according to claim 3 or 4, characterized in that the activated carbon is in the form of granules and the montmorillonite is in powder form.

6. Process according to claim 2, characterized in that the adsorbent mixture contains approximately 50 % by weight of silica gel and approximately 50 % by weight of montmorillonite.

7. Process according to claim 2 or 6, characterized in that the montmorillonite consists of 40 to 60 % by weight of powder and 60 to 40 % by weight of granules.